# EUROPEAN PATENT SPECIFICATION

(11) **EP 2 231 866 B1**
(45) Date of publication and mention of the grant of the patent: **26.11.2014**
(21) Application number: 08866453.7
(22) Date of filing: 31.12.2008
(51) Int. Cl.: C12Q 1/68, C12Q 1/14, C12N 15/11

(54) **METHODS AND OLIGONUCLEOTIDES FOR DETECTION OF MASTITIS CAUSING BACTERIA**
VERFAHREN UND OLIGONUKLEOTIDE FÜR DEN NACHWEIS VON MASTITIS VERURSACHENDEN BAKTERIEN
PROCÉDÉS ET OLIGONUCLÉOTIDES POUR LA DÉTECTION DE BACTÉRIES PROVOQUANT UNE MASTITE

(30) Priority: 31.12.2007 FI 20075976; 31.12.2007 US 17990 P
(43) Date of publication of application: 29.09.2010
(73) Proprietor: Thermo Fisher Scientific Oy, 01620 Vantaa (FI)
(72) Inventor: HOLOPAINEN, Jani, FI-01390 Vantaa (FI); KOSKINEN, Mikko, FI-02180 Espoo (FI)
(74) Representative: Karvinen, Leena Maria
(86) International application number: PCT/FI2008/050795
(87) International publication number: WO 2009/083656

(56) References cited:
- EP-A1- 1 770 171
- WO-A2-01/23604
- WO-A2-98/20157
- US-A- 5 849 488
- ABDEL HAMEID KARIMA GALAL ET AL: "Multiplex PCR protocol for the diagnosis of cow udder infection with Staphylococcus aureus", ANIMAL SCIENCE PAPERS AND REPORTS, POLISH SCIENTIFIC PUBLISHERS, WARZSAW, PL, vol. 22, no. 4, 1 January 2004 (2004-01-01), pages 679-685, XP009096125, ISSN: 0860-4037
- MARTINEAU F ET AL: "Development of a PCR assay for identification of staphylococci at genus and species levels", JOURNAL OF CLINICAL MICROBIOLOGY, AMERICAN SOCIETY FOR MICROBIOLOGY, WASHINGTON, DC, US, vol. 39, no. 7, 1 July 2001 (2001-07-01), pages 2541-2547, XP003007591, ISSN: 0095-1137, DOI: DOI:10.1128/JCM.39.7.2541-2547.2001
- SABAT ET AL: "New Method for Typing Staphylococcus aureus Strains: Multiple-Locus Variable-Number Tandem Repeat Analysis of Polymorphism and Genetic Relationships of Clinical Isolates", JOURNAL OF CLINICAL MICROBIOLOGY, AMERICAN SOCIETY FOR MICROBIOLOGY, WASHINGTON, DC, US, vol. 41, no. 4, 1 April 2003 (2003-04-01), pages 1801-1804, XP002275199, ISSN: 0095-1137, DOI: DOI:10.1128/JCM.41.4.1801-1804.2003
- GILLESPIE B E ET AL: "Simultaneous Detection of Mastitis Pathogens, Staphylococcus aureus, Streptococcus uberis, and Streptococcus agalactiae by Multiplex Real-Time Polymerase Chain Reaction", JOURNAL OF DAIRY SCIENCE, AMERICAN DAIRY SCIENCE ASSOCIATION, US, vol. 88, no. 10, 1 October 2005 (2005-10-01), pages 3510-3518, XP026956722, ISSN: 0022-0302 [retrieved on 2005-10-01]
- KARIMA GALAL ABDEL HAMEID ET AL.: 'Multiplex PCR protocol for the diagnosis of cow udder infection with Staphylococcus aureus' ANIMAL SCIENCE PAPERS AND REPORTS vol. 22, no. 4, 2004, pages 679 - 685, XP009096125
- MARTINEAU F. ET AL.: 'Development of a PCR Assay for Identification of Staphylococci at Genus and Species Levels' JOURNAL OF CLINICAL MICROBIOLOGY vol. 39, no. 7, July 2001, pages 2541 - 2547, XP003007591
- DATABASE REGISTRY [Online] RN 364114-06-7, 2009 XP003025526 Retrieved from STN & WO 01 70955 A2 (ELITRA PHARMACEUTICALS, INC.) 27 September 2001 -& DATABASE REGISTRY [Online] RN 364125-09-7, 2009 XP003025527 Retrieved from STN
- MASON W.J. ET AL.: 'Multiplex PCR Protocol for the Diagnosis of Staphylococcal Infection' JOURNAL OF CLINICAL MICROBIOLOGY vol. 39, no. 9, 2001, pages 3332 - 3338, XP002272884

### SEQUENCE LISTING

<110> Finnzymes Oy
<120> Methods and oligonucleotides for detection of mastitis causing bacteria
<130>
<160> 11
<170> PatentIn version 3.1
<210> 1
   <211> 21
   <212> DNA
   <213> Artificial sequence
<220>
   <223> Oligonucleotide primer or probe
<400> 1
   ttcaactgaa gcaacacctt c 21
<210> 2
   <211> 25
   <212> DNA
   <213> Artificial sequence
<220>
   <223> Oligonucleotide primer or probe
<400> 2
   cacttgtatt aaccgcttga ttaac 25
<210> 3
   <211> 25
   <212> DNA
   <213> Artificial sequence
<220>
   <223> Oligonucleotide primer or probe
<400> 3
   tacttggatt aaccgcttga ctaac 25
<210> 4
   <211> 21
   <212> DNA
   <213> Artificial sequence
<220>
   <223> Oligonucleotide primer or probe
<400> 4
   aactccagaa cgtgattctg a 21
<210> 5
   <211> 21
   <212> DNA
   <213> Artificial sequence
<220>
   <223> Oligonucleotide primer or probe
<400> 5
   tgatttgacc acgttcaaca c 21
<210> 6
   <211> 27
   <212> DNA
   <213> Artificial sequence
<220>
   <223> Oligonucleotide primer or probe
<400> 6
   tgaatcagct ccacagagta cagatgc 27
<210> 7
   <211> 25
   <212> DNA
   <213> Artificial sequence
<220>
   <223> Oligonucleotide primer or probe
<400> 7
   cattcatgat gccagttgag gacgt 25
<210> 8
   <211> 3090
   <212> DNA
   <213> Staphylococcus aureus subsp. aureus strain MRSA252
<400> 8
   atgaatatga agaaacaaga aaaacacgca attcgtaaaa aatcgattgg cgtggcttca     60
   gtgcttgtag gtacgttaat cggttttgga ctactcagca gtaaagaagc agatgcaagt    120
   gaaaatagta tgacacaaac ggaaaatacg agtaatgaga gcaaaagtaa tgatccaagt    180
   agcgttaatg ctgcacctaa aacagacaac acaaacgtga gtgattctaa tacaacgaca    240
   aacactaata gtgacgaaac gaatgtagcg caaaatccag cacaacagga aacgacacaa    300
   tcagcatcaa caaatgcaac tacagaagaa acaccggtaa ctggtgaagt tactactacg    360
   gcaacgaatc aagctaatac accggcaaca actcaatcaa gcaatacaaa tgcggaagaa    420
   tcagtgaatc aaacaagtaa tgaaacgact tctaatgata ctaatacagt atcatctgta    480
   aattcacctc aaaattctac aaatgcggaa aatgtttcaa caacgcaaga catttcaact    540
   gaagcaacac cttcaaacaa tgaatcagct ccacagagta cagatgcaag taataaagat    600
   gtagttaatc aagcggttaa tacaagtgcg cctagaatga gagcatttag tttagcggct     660
   gtagctgcag atgcaccggc tgctggcaaa gatattacga atcagttgac gaatgtgaca     720
   gttggtattg actctggaga tacagtttat ccgcaccaag caggctatgt caaactgaat     780
   tatgggttct cagtaccaaa tgaggctgtt caaggtgaca cattcaaaat aactgtgccc     840
   aaagaattaa acttaaatgg tgtaacttca actgctaaag tgccaccaat tatggccgga     900
   gatcaagtat tggcaaatgg tgtaatcgat agtgatggta atgttattta tacatttaca     960
   gactatgtaa atactaaaga tgatgttaaa gcaactttga ccatgcccgc ttatattgac    1020
   cctgaaaatg ttacaaagac aggtaatgtg acattggcta ctggcatagg tagtacaaca    1080
   gcaaacaaaa cagtattagt agattatgaa aaatatggta agttttataa cttatctatt    1140
   aaaggtacaa ttgaccaaat cgataaaaca aataatacgt atcgtcagac aatttatgtc    1200
   aatccaagtg gagataacgt tattgcgccg gttttaacag gtaatttaaa accaaatacg    1260
   gatagtaatg cattaataga tgcacaaaat actagtatta aagtatataa agttgataat    1320
   gcatcagact tgtctgaaag ttattatgtg aatccagata actttgaaga tgtcactgat    1380
   agtgtgaata ttacattccc aaatccaaat caatataaag tagagttcaa tacgcctgat    1440
   gatcaaataa caacaccata tattgtagtt gttaatgggc atattgatcc taatagtaaa    1500
   ggtgatttag ctttacgttc aactttatat ggatataatt cgaatataat ttggcgatca    1560
   atgtcatggg ataatgaagt agcatttaat aacggatcag gttctggtga cggtatcgat    1620
   aaacctgttg ttcctgaaca acctgatgag ccgggtgaaa ttgaaccaat tccagaggat    1680
   tcagattctg acccaggttc agatagtggt tcagattctg gcagcgattc taattcagat    1740
   agcggttcag attcgggtag tgattctaca tcagatagtg gttcagattc agcgagcgat    1800
   tcagattcag caagtgattc agattcagcg agtgattcag attcagcaag tgattcagat    1860
   tcagcgagtg attcagattc agcaagtgat tcagattcag cgagtgattc agattcaacg    1920
   agtgattcag attcaacgag tgattccgat tcagcaagcg attcagattc agatagtgac    1980
   tcagactcag acagcgattc agattcagat agtgactcag actcagatag cgattcagat    2040
   tccgacagtg actcggattc agatagcgat tccgactcag acagtgactc agattcagat    2100
   agcgactcag actcagaaag cgactcagat tcagacagcg attccgactc agatagtgat    2160
   tcagattcag acagcgactc agacagtgtt tcagattcag acagtgactc agactcggat    2220
   agtgaatcag actcagacag cgactcagat tcagacagcg actcagactc ggatagtgaa    2280
   tcagactcag acagtgactc agactcagac agcgactcag actcagatag cgactcagat    2340
   tcagatagcg attcagactc agacagcgac tcagactcag atagcgattc cgactcggat    2400
   agcgactcag actcagatag cgattccgac tcggatagcg attcagaatc agatagtgac   2460
   tcagattcag atagcgattc cgactcagat agcgactcag attccgacag cgattcagat   2520
   tcagacagtg atttagactc agacagtgac tcggattcag atagcgattc cgactcagac   2580
   agtgactcgg attccgacag cgattcagat tcagacagtg atttagactc agacagtgac   2640
   tcggattcag atagcgattc cgactcagat agtgcctcag attcagatag cgactcagac   2700
   tcagacagcg attcagaatc agatagtgac tcagattcag atagcgattc cgactcagac   2760
   agtgcctcag attcagatag tgactcggat tcagcgagtg attccgattc agattcaacg   2820
   agtgacacag gatcagataa cgactcagac tcagaaagtg attcgaatag cgattccgat   2880
   tcaggttcta acaataatgt agtgccgcct aattcaccta aaagtggtac taatgcttca   2940
   aataaaaatg aggctaaaga aagtaaagaa ccattaccag atacaggttc tgaagatgaa   3000
   gctaatacgt cactaatttg gggattatta gcatcattag gttcattact acttttcaga   3060
   agaaaaaaag aaaataaaga taagaaataa                                     3090
<210> 9
   <211> 1185
   <212> DNA
   <213> Staphylococcus epidermidis ATCC 12228
<400> 9
   atggcaaaag aaaaatttga tcgctcaaaa gaacatgcca atattggtac tatcggtcac      60
   gttgaccatg gtaaaacaac tttaacagct gctatcgcaa ctgtattagc taaaaatggt     120
   gacactgttg cacaatcata cgatatgatt gacaacgctc cagaagaaaa agaacgtggt     180
   attacaatca atactgcaca tatcgaatac caaactgaca aacgtcacta tgctcacgtt     240
   gactgcccag gacacgctga ctatgttaaa aacatgatca ctggtgcagc tcaaatggac     300
   ggcggtatct tagttgtatc tgctgctgac ggtccaatgc cacaaactcg tgaacacatc     360
   ttattatcac gtaacgttgg tgtaccagca ttagttgtat tcttaaacaa agttgacatg     420
   gtagacgacg aagaattatt agaattagtt gaaatggaag ttcgtgactt attaagcgaa     480
   tatgacttcc caggtgacga tgtacctgta atcgctggtt ctgcattaaa agcattagaa     540
   ggcgatgctg aatacgaaca aaaaatctta gacttaatgc aagcagttga tgattacatt     600
   ccaactccag aacgtgattc tgacaaacca ttcatgatgc cagttgagga cgtattctca     660
   atcactggtc gtggtactgt tgctacaggc cgtgttgaac gtggtcaaat caaagttggt     720
   gaagaagttg aaatcatcgg tatgcacgaa acttctaaaa caactgttac tggtgtagaa     780
   atgttccgta aattattaga ctacgctgaa gctggtgaca acatcggtgc tttattacgt     840
   ggtgttgcac gtgaagacgt acaacgtggt caagtattag ctgctcctgg ttctattaca     900
   ccacacacaa aattcaaagc tgaagtatac gtattatcta aagatgaagg tggacgtcac     960
   actccattct tcactaacta tcgcccacaa ttctatttcc gtactactga cgtaactggt    1020
   gttgtaaact taccagaagg tacagaaatg gttatgcctg gcgacaacgt tgaaatgaca    1080
   gttgaattaa tcgctccaat cgctatcgaa gacggaactc gtttctcaat tcgtgaaggt    1140
   ggacgtactg ttggatcagg cgttgtaact gaaatctttg aataa                    1185
<210> 10
   <211> 95
   <212> DNA
   <213> Staphylococcus aureus
<400> 10
   ttcaactgaa gcaacacctt caaacaatga atcagctcca cagagtacag atgcaagtaa      60
   taaagatgta gttaatcaag cggttaatac aagtg                                 95
<210> 11
   <211> 110
   <212> DNA
   <213> Staphylococcus sp.
<400> 11
   aactccagaa cgtgattctg acaaaccatt catgatgcca gttgaggacg tattctcaat     60
   cactggtcgt ggtactgttg ctacaggccg tgttgaacgt ggtcaaatca              110

## Description

The present invention relates to the field of polymerase chain reaction (PCR) based diagnostic assays. More specifically, the present invention provides a PCR based method for detecting mastitis causing bacteria, particularly *Staphylococcus aureus* and coagulase negative staphylococci. The present invention also provides oligonucleotide primers and probes for use in said method.

### BACKGROUND OF THE INVENTION

Mastitis, i.e. inflammation of a cow's mammary gland, leads to a significant decrease in milk yield and quality and represents a major problem to the dairy industry world-wide. In acute mastitis, the udder is commonly infected by one primary pathogen. In many countries, staphylococci are the most common agents of mastitis: for example in Finland, in 2001, more than one-half of the intramammary infections were caused by either coagulase negative staphylococci (CNS) or *Staphylococcus aureus* (Pitkälä et al. 2004). In contrast to CNS and *S. aureus* responsible for human infections, the bovine mastitis-related staphylococci can often be successfully treated with beta-lactam antibiotics (e.g., penicillin). Resistance of CNS and *S. aureus* to beta-lactam antibiotics is caused by an enzyme known as beta-lactamase. The 'blaZ' gene is responsible for production of beta-lactamase and CNS and *S. aureus* harbouring the gene are penicillin resistant, while strains lacking the gene can be successfully treated with beta-lactam antibiotics.

Bacterial culturing on blood-esculin-agar remains the gold standard for diagnosing intramammary infections. When correctly performed, culturing can provide a very specific and sensitive means for mastitis pathogen testing, as well as testing their capability to produce beta-lactamase. However, culturing is notoriously slow, with laboratory turn-around times being routinely up-to 48 hours. Consequently, the current testing schemes often fail to guide the treatment of an animal. Instead, due to the long analyses times, antibiotic treatment is routinely started for cows exhibiting symptoms of an acute infection, prior to receiving the test results (Sandholm 1995). It follows, that in a significant proportion of all mastitis cases the treatment may be unnecessary either due to resistance of the pathogen to the primary choice of antibiotics (penicillin) or due to the fact that in some 20% of inflammations the infection is caused by something else than bacteria sequestered in the udder (Sandholm 1995). This practise leads to much unnecessary antibiotic use, as well as significant economical losses to the farmers, through added drug costs and discarded milk.

PCR (polymerase chain reaction) assays and its derivatives, such as qPCR assays (quantitative polymerase chain reaction, also known as real-time polymerase chain reaction), aiming to amplify, identify and quantify species-specific nucleic acids have become widely used in routine microbial testing. PCR methods have potential to provide significant analysis time savings. Hence, PCR-based tests would seem to hold much promise for mastitis testing (see, e.g.,Gillespie & Oliver 2005).

### Challenges in the Development of a PCR Method for Mastitis Testing

The most important mastitis pathogens include at least the following: *Staphylococcus aureus,* Coagulase Negative Staphylococci (CNS; a species group comprising over 30 genetically closely related but difficult-to-identify species), *Streptococcus dysgalactiae, Streptococcus agalactiae, Streptococcus uberis, Escherichia coli, Enterococcus* spp., *Corynebacterium bovis, Arcanobacterium pyogenes, Klebsiella* spp., *Lactococcus lactis* and *Serratia* spp.

In the development of PCR assays, one of the most important factors is to locate oligonucleotide sequences that enable reliable species-specific amplification, detection and quantification. It is of utmost importance that a given set of oligonucleotides, designed to amplify e.g., *Staphylococcus aureus,* does not cross-react with DNA originating from any other species possibly present in the sample matrix. Finding such sequences can be far from trivial, at least for the following reasons: 1) many of the species are relatively closely related, making it challenging to locate sequences that are unique for each species; 2) mastitis pathogen strains originating from a single species can be genetically diverged, making it difficult to locate sequences that would enable equally efficient amplification of all strains within a species: 3) the sample matrix in mastitis diagnostics is commonly raw milk, containing a host of PCR inhibitors. Hence, in order to effectively amplify pathogen DNA from all milk samples requires oligonucleotide design enabling high PCR efficiency (optimally as close to 100% as possible); 4) the number of pathogens causing mastitis is large (see above) and a mastitis testing method should optimally identify all of them. Having one PCR reaction per species can be cumbersome, since the number of samples tested is typically large. It would be optimal to detect multiple species within one reaction. In a PCR setting the most obvious alternative is 'multiplex' PCR amplification. In multiplex PCR, several oligonucleotide sets, each designed to amplify one species/species group, are included in the same reaction vessel and each oligonucleotide set is used to amplify its respective pathogen DNA during the same PCR reaction. Multiplex PCR presents a challenge for quantitation of the pathogen DNA (qPCR): the different amplicons compete for the same PCR reaction components (eg. DNA polymerase and MgCl2) and this can compromise the quantitative nature of the reaction between and, especially, quantitative comparisons between samples.

The present inventors have now located DNA sequence regions that are well suited for specific and sensitive amplification and quantification of mastitis pathogens, particularly *Staphylococcus aureus* and coagulase negative staphylococci, using template DNA extracted from raw bovine milk. Optimal primers and quantitative PCR probes have been designed and validated for identification and quantification of the mastitis pathogens. A collection of hundreds of bacterial strains has been used in assessing the specificity of the oligonucleotide design.

Prior PCR assays for the detection of *Staphylococcus aureus* and Staphylococci are disclosed by Sakai et al, 2004, Maes et al, 2002 and Martineau et al, 2001 as well as in U.S. Patent No. 5,702,895. Other prior art related to this particular technical field are: U.S. Patent No. 5,849,488 disclosing a method for detecting presence of DNA sequences in a milk sample; U.S. Patent No. 6,720,160 disclosing a method for simultaneous detection of plurality of different indicators of mastitis; and Riffon et al, 2001, disclosing development of a PCR-test for identification of major pathogens in bovine mastitis.

Further, Galal Abdel Hameid et al., 2004, (Animal Science Papers and Reports, vol. 22, No. 4, pages 679-685) disclose a method for identification of Staphylococci strains in milk in association with mastitis, comprising extracting DNA from bacteria isolated from milk samples and performing a multiplex PCR with primers to detect a region of the 16S rRNA conserved among Staphylococci and primers to detect *S. aureus* cflA gene. In WO 98/20157, the use of the *tuf* gene to design common primers for the identification of Staphylococcus species is disclosed, and Martineu et al., 2001, (Journal of Clinical Microbiology, vol. 39, No. 7, pages 2541-2547) disclose genus-specific primers and probe from the *tuf* gene sequence. Sabat et al., 2003, disclose a PCR-based methodology for typing *S. aureus* strains where five staphylococcal VNTR loci (*sdr, clfA, clfB, ssp,* and *spa*) were subjected to analysis.

### DETAILED DESCRIPTION OF THE INVENTION

The invention is defined by the claims.

The present specification discloses a method for determining the presence of mastitis causing bacteria in a milk sample comprising the steps of:
a) isolating nucleic acid from a milk sample or a sample derived from milk, preferably as described in Gillespie and Oliver 2005;
b) contacting the isolated nucleic acid obtained in step a) in a polymerase chain reaction mix with primers specifically hybridizing with the nucleic acid sequence of clumping factor gene of *Staphylococcus aureus* and primers specifically hybridizing with the nucleic acid sequence of elongation factor Tu gene of *Staphylococcus* spp.; and
c) performing a polymerase chain reaction with a reaction mix obtained from step b), wherein the detection of desired hybrids formed between said primers and said isolated nucleic acid indicates the presence of *Staphylococcus aureus* and/or *Staphylococcus* spp. in said sample. The desired hybrids are preferably detected by determining the presence of a PCR-product generated by extension of said primers while said primers are specifically hybridized to the isolated nucleic acid, i.e. said isolated nucleic acid is used as a template for the extension product. In other words, a polymerase chain reaction is performed with a reaction mix obtained from step b) so that the sequences of said clumping factor gene and/or elongation factor Tu gene are specifically amplified, if said sequences are present in the sample. Thereafter, the presence of the amplified nucleid acid sequences is detected, wherein the presence of amplified nucleic acid sequence from both clumping factor gene and elongation factor Tu gene is indicative of the presence of *Staphylococcus aureus* in the sample, and wherein the presence of amplified nucleic acid sequence from only elongation factor Tu gene is indicative of the presence of *Staphylococcus* spp. in said sample.

Thus, the preliminary diagnostic results of the above method are interpreted in the following way: (1) if the test is positive for both *Staphylococcus aureus* and *Staphylococcus* spp. (i.e. coagulase negative staphylococci), then the cause of mastitis is likely *Staphylococcus aureus,* or (2) if the test is negative for *Staphylococcus aureus* but positive for *Staphylococcus* spp., then the cause of mastitis is likely *Staphylococcus* spp. other than *Staphylococcus aureus.*

In one of the most preferred embodiment of the present invention, said method is performed as a real-time polymerase chain reaction for which several commercial kits are available. Thus, in step b) said isolated nucleic acid obtained in step a) may further be contacted with a probe specifically hybridizing with the nucleic acid sequence of clumping factor gene of *Staphylococcus aureus* and/or a probe specifically hybridizing with the nucleic acid sequence of elongation factor Tu gene of *Staphylococcus* spp. The primers of the method can also be used in a multiplex PCR-reaction comprising further primer pairs and probes for simultaneous detection of other bacteria in a milk sample. Such other bacteria may be selected from the group consisting of: *Streptococcus dysgalactiae, Streptococcus agalactiae, Streptococcus uberis, Escherichia coli, Enterococcus* spp., *Corynebacterium bovis, Arcanobacterium pyogenes, Klebsiella* spp. (such as *Klebsiella pneumoniae* and *Klebsiella oxytoca*), *Lactococcus lactis, Serratia* spp. (such as *Serratia marcescens*) and *Peptostreptococcus indolicus.*

In the present invention, the primers specifically hybridize with the nucleic acid sequence of clumping factor gene of *Staphylococcus aureus* so that the amplification product obtained comprises at least part of the 95bp long stretch in said clumping factor gene corresponding to nucleotides 534-628 in SEQ ID NO:8. In elongation factor Tu gene of *Staphylococcus* spp. the primers amplify at least part of the 119bp long amplicon in said elongation factor Tu gene corresponding to nucleotides 603-721 in SEQ ID NO:9.

In order to decide the correct treatment for an animal having mastitis, the method of the invention preferably comprises a further or simultaneous step of detecting the presence of β-lactamase production or a β-lactamase gene encoding resistance to β-lactam antibiotics in said sample. If the result of this step is positive, the animal should not be treated with β-lactam antibiotics such as penicillin.

The present invention also provides oligonucleotide primers specifically hybridizing with the nucleic acid sequence of clumping factor gene of *Staphylococcus aureus,* said primers having the sequence selected from the group consisting of SEQ ID NO:1, SEQ ID NO:2 and SEQ ID NO:3.

The present invention also provides oligonucleotide primers specifically hybridizing with the nucleic acid sequence of elongation factor Tu gene of *Staphylococcus* spp., said primers having the sequence selected from the group consisting of: SEQ ID NO:4 and SEQ ID NO:5.

For real-time polymerase chain reactions, the invention provides probes specifically hybridizing with the nucleic acid sequence of clumping factor gene of *Staphylococcus aureus* and with the nucleic acid sequence of elongation factor Tu gene of *Staphylococcus* spp., said probes having the sequences selected from the group consisting of SEQ ID NO:6 and SEQ ID NO:7, respectively.

The present invention is also directed to the use of a primer or probe specifically hybridizing with the nucleic acid sequence of clumping factor gene of *Staphylococcus aureus* (and having a sequence as defined in the Sequence Listing) for the detection of the presence of *Staphylococcus aureus* in a sample.

The present invention is further directed to the use of a primer or probe specifically hybridizing with the nucleic acid sequence of elongation factor Tu gene of *Staphylococcus* spp. (and having a sequence as defined in the Sequence Listing) for the detection of the presence of *Staphylococcus* spp. in a sample.

The oligonucleotides as disclosed herein are short sequences of nucleotides (such as RNA or DNA, preferably DNA), typically with twenty-five to thirty or fewer bases. However, automated synthesizers allow the synthesis of oligonucleotides up to 160 to 200 bases and the present oligonucleotides may be elongated to add, e.g., a restriction enzyme cleavage site, to the oligonucleotide. The typical length of the primers is preferably 18-26, more preferably 20-24 nucleotides.

The present invention also provides kits for the detection of the presence of mastitis causing bacteria in a milk sample. Such a kit may comprise a pair of primers specifically hybridizing with the nucleic acid sequence of clumping factor gene of *Staphylococcus aureus* and a pair of primers specifically hybridizing with the nucleic acid sequence of elongation factor Tu gene of *Staphylococcus* spp.

The kit may further comprise a probe specifically hybridizing with the nucleic acid sequence of clumping factor gene of *Staphylococcus aureus* and/or a probe specifically hybridizing with the nucleic acid sequence of elongation factor Tu gene of *Staphylococcus* spp. Such primer pairs and probes are described above and in the Examples below.

Preferably, said kit comprises means for a real-time polymerase chain reaction, such as labelled probes, polymerase enzymes, buffers and nucleotides.

It is well-known in the art that the sequences of same gene vary somewhat in strains of a bacterial species and, thus, the sequence of clumping factor gene of *Staphylococcus aureus* is not 100% identical in all strains of the species. The same applies to the elongation factor Tu gene of *Staphylococcus* spp. However, it is clear from the description herein, particularly from the Example below, that a person skilled in the art would recognize the sequence of a clumping factor gene of *Staphylococcus aureus* or a elongation factor Tu gene of *Staphylococcus* spp. in any related strain based on the similarity and homology of these gene sequences.

Herein the term "specifically hybridizing" means complementary hybridization between an oligonucleotide and a target sequence. The term "specifically" refers to the specificity shown by the complementary hybridization, which allows for minor mismatches between the oligonucleotide and the sequence that may not jeopardize the annealing for detection of hybridization signals.

The present invention is further described in the following example, which is not intended to limit the scope of the invention.

### EXAMPLE

### Materials and Methods

### Bacterial Strains

The monoculture bacterial strains used in specificity and sensitivity testing of the oligonucleotide primers and probes contained bacteria from various countries. A total of 424 different samples were tested. The more detailed information about bacterial strains is presented in Table 1.

### Isolation of template-DNA

Bacterial cells from monocultures were suspended in glycerol. DNA extraction protocol contained three solutions: PPT {69% Trition-X (20%), 9% Tween (100%), 2% ProtK and 20% DTT (200nM)}, MURLYL {60% mutanolysin (1U/µl), 15% lysozyme (20µg/µl), 5% RNAse (20µg/µl), 20% lysostaphin (1U/µl) and CHEP II {94% Chelex (50% w/v)}, 6% protK. Equal volumes (100µl) of bacterial solution and PPT solution was pipetted into a 1.5 mL microcentrifuge tube and vortexed before 5 min incubation at 55°C. The sample was centrifuged for 5min at 14000 rfc. The supernatant was discarded and bacterial pellet was incubated for 5 min at 95°C in order to inactivate the protK. The bacterial pellet was suspended in 20 µl of MURLYL solution, and then vortexed and incubated for 30min at 37 °C. After adding 25µl of CHEP II solution into the tube, the sample was vortexed and incubated for 5 min at 55 °C and then for 5 min at 95 °C. After the sample was centrifuged for 5min at 14000 rfc the upper, DNA containing, bright layer was removed from the tube.

### Oligonucleotide Primers and Probes

*S. aureus* oligos are designed to amplify 95bp long amplicon from S. *aureus* clumping factor gene (clfA). The clfA gene encodes a fibrinogen-binding protein. In *Staphylococcus aureus* subsp. *aureus,* strain MRSA252, the coding sequence of clfA having Accession No. BX571856 is as set worth in SEQ ID NO:8. The corresponding gene sequence naturally vary in related strains. In SEQ ID NO:8, said 95bp long amplicon corresponds to nucleotides 534-628.

*Staphylococcus* spp. oligos are designed to amplify 119bp long amplicon from *tuf* gene, which encodes the elongation factor Tu. In *Staphylococcus epidermidis* ATCC 12228 the coding sequence of *tuf* gene having Accession No. NC_004461 is as set worth in SEQ ID NO:9. The corresponding sequence naturally vary in related strains. In SEQ ID NO:9, said 119bp long amplicon corresponds to nucleotides 603-721.

*Tuf* and clfA gene oligosequences were obtained from GeneBank (www.ncbi.nih.gov). In addition, four *S. aureus* strains (173, 2401, 4289, and 5341) were sequenced.

Primers and probes were designed using Primer3 program (available in the internet at http://frodo.wi.mit.edu/cgibin/primer3/primer3_www.cgi). At first, the primers and probes were designed for single qPCR reactions, but then oligos were multiplexed, since their sensitivity and specificity were discovered to be adequately high for multiplex reactions. Designed oligos were divided into three different multiplex primer mixes. Multiplex primer mix 1 contains oligos for *S. aureus, C. bovis* and Enterococcus spp. detection. Multiplex primer mix 2 contains oligos for Staphylococcus spp., *Str. uberis* and *Str. agalactiae* detection, and multiplex primer mix 3 contains oligos for blaZ gene, *Str. dysgalactiae* and *E. coli* detection. Designed primer and probe sequences for *S. aureus* and *Staphylococcus* spp. detection are disclosed in Table 2.

### Multiplex qPCR Assay

The F-450 2x DyNAmo probe Master Mix (Finnzymes Oy, Espoo, Finland) was used for the multiplex real-time PCR assay. The F-450 2x DyNAmo probe Master Mix contains hot start *Tbr* polymerase, optimized PCR buffer, MgCl₂ and dNTP mix including dUTP. Each reaction contained 10µl F-450 Master Mix, 500nM each primer, 250nM each dual-labeled probe, and sterile water to bring the final volume to 20µl. The Chromo4 real-time PCR detection system (MJ Research) was used with following program: 95°C for 10 min followed by 40 repeats of 95°C for 5 s and 60°C for 1 min and fluorescence measurement. After 40 cycles repeat samples were incubated 10 s at 10°C.

### Determination of sensitivity

Sensitivity and specificity of real-time PCR reactions were determined essentially as described in Gillespie and Oliver, 2005.

### Results

While determining the sensitivity and specificity of the *S. aureus* and *Staphylococcus* oligos in single qPCR reactions, the threshold line was set to 0.020 (probes contained 6-FAM dye). A sample was determined positive if its fluorescence rose above the threshold line before cycle 35, i.e. its Ct (Cycle threshold) value was under 35. Samples having Ct values over 35 scored negative. Thus, the sensitivities and specificities were 100% in both cases.

Sensitivity for *S. aureus* oligos: 146 / (146 + 0) * 100% = 100%
Specificity for *S. aureus* oligos: 278 / (278 + 0) * 100% = 100%

Sensitivity for Staphylococcus oligos: 235 / (235 + 0) * 100% = 100%
Specificity for Staphylococcus oligos: 189 / (189 + 0) * 100% = 100%

**Table 1. Sample strains used in specificity and sensitivity testing.**

| **Bacterial species** | **Origin of strains** | **number of strains** |
|---|---|---|
| *S. aureus* | Finland, Italy, Norway, Portugal, Canada, USA | 146 |
| CNS | Finland, Italy, Norway, Portugal, Canada | 89 |
| *Str. agalactiae* | USA, Canada, Italy, Norway, Portugal | 8 |
| *Str. dysgalactiae* | USA, Canada, Italy, Norway, Portugal | 31 |
| *Str. uberis* | Canada, Italy, Norway, Portugal | 26 |
| *Str. bovis* | Finland | 5 |
| *E. coli* | Canada, Italy, Norway, Portugal | 45 |
| *C. bovis* | Portugal | 8 |
| *A.pyogenes* | Norway | 3 |
| Enterococcus spp. | USA, Italy, Finland | 37 |
| Klebsiella spp. | USA, Canada, Finland | 16 |
| Serratia spp. | Finland, Canada | 5 |
| Pseudomonas spp. | Finland | 1 |
| Citrobacter spp. | Canada | 1 |
| Enterobacter spp. | Finland, Canada | 3 |

**Table 2. Primer and probe sequences for S. aureus and Staphylococcus spp.**

| **Primer and probe sequences** | | |
|---|---|---|
| ***S. aureus*** | | |
| forward | TTCAACTGAAGCAACACCTTC | SEQ ID NO:1 |
| reverse | CACTTGTATTAACCGCTTGATTAAC | SEQ ID NO:2 |
| reverse2 | TACTTGGATTAACCGCTTGACTAAC | SEQ ID NO:3 |
| probe | TGAATCAGCTCCACAGAGTACAGATGC | SEQ ID NO:6 |

| ***Staphylococcus* spp.** | | |
|---|---|---|
| forward | AACTCCAGAACGTGATTCTGA | SEQ ID NO:4 |
| reverse | TGATTTGACCACGTTCAACAC | SEQ ID NO:5 |
| probe | CATTCATGATGCCAGTTGAGGACGT | SEQ ID NO:7 |

**Table 3. Amplicons amplified in target strains.**

| |
|---|
| *S. aureus* amplicon in clumping factor gene: |
| |
| *Staylococcus* sp. amplicon in elongation factor Tu gene: |
| |

### REFERENCES

Gillespie, B. E., and S. P. Oliver. 2005. Simultaneous detection of Staphylococcus aureus, Streptococcus agalactiae and Streptococcus uberis in milk by multiplex real-time polymerase chain reaction. J. Dairy Sci. 88:3510-3518.
Maes, N., Magdalena, J., Rottiers, S., De Gheldre, Y., and Struelens M.J. 2002. Evaluation of a Triplex PCR Assay To Discriminate Staphylococcus aureus from Coagulase-Negative Staphylococci and Determine Methicillin Resistance from Blood Cultures. Journal of Clinical Microbiology, 40(4):1514-1517.
Martineau, F., Picard, F.J., Ke, D., Paradis, S., Roy, P.H., Ouellette, M., and Bergeron, M.G. 2001. Development of a PCR Assay for Identification of Staphylococci at Genus and Species Levels. Journal of Clinical Microbiology, 39(7):2541-2547.
Pitkälä, A., Haveri, M., Pyörälä, S., Myllys, V. And Honkanen-Buzalski, T. 2004, Bovine Mastitis in Finland 2001 - Prevalence, Distribution of bacteria, and Antimicrobial resistance. J. Dairy Sci. 87:2433-2441
Riffon, R., Sayasith, K., Khalil, H., Dubreuil, P., Drolet, M., and Lagace, J. 2001, Development of a rapid and sensitive test for identification of major pathogens in bovine mastitis by PCR. Journal of Clinical Microbiology 39(7):2584-2589.
Sakai, H., Procop, G.W., Kobayashi, N., Togawa, D., Wilson, D.A., Borden, L., Krebs, V., and Bauer T.W. 2004. Simultaneous Detection of Staphylococcus aureus and Coagulase-Negative Staphylococci in Positive Blood Cultures by Real-Time PCR with Two Fluorescence Resonance Energy Transfer Probe Sets. Journal of Clinical Microbiology, 42(12):5739-5744.
Sandholm, M., Honkanen-Buzalski, T., Kaartinen, L. and Pyörälä, S. 1995, the Bovine Udder and Mastitis. Gummerus Kirjapaino OY, Jyväskylä.

## Claims

1. Method for determining the presence of mastitis causing bacteria in a milk sample comprising the steps of:
a) isolating nucleic acid from a milk sample or a sample derived from milk;
b) contacting the isolated nucleic acid obtained in step a) in a polymerase chain reaction mix with primers specifically hybridizing with the nucleic acid sequence of clumping factor gene of *Staphylococcus aureus* and amplifying at least part of the 95bp long amplicon in said clumping factor gene corresponding to nucleotides 534-628 in SEQ ID NO:8 and with primers specifically hybridizing with the nucleic acid sequence of elongation factor Tu gene of *Staphylococcus* spp. and amplifying at least part of the 119bp long amplicon in said elongation factor Tu gene corresponding to nucleotides 603-721 in SEQ ID NO:9; and
c) performing a polymerase chain reaction with a reaction mix obtained from step b) so that the sequences of said clumping factor gene and elongation factor Tu gene are specifically amplified, if said sequences are present in the sample;
d) detecting the presence of the amplified nucleid acid sequences, wherein the presence of amplified nucleic acid sequence from both clumping factor gene and elongation factor Tu gene is indicative of the presence of *Staphylococcus aureus* in the sample, and wherein the presence of amplified nucleic acid sequence from only elongation factor Tu gene is indicative of the presence of *Staphylococcus* spp. in the sample.

2. The method according to claim 1, wherein in step b) said isolated nucleic acid obtained in step a) is further contacted with a probe specifically hybridizing with said nucleic acid sequence of clumping factor gene of *Staphylococcus aureus* and/or a probe specifically hybridizing with said nucleic acid sequence of elongation factor Tu gene of *Staphylococcus* spp.

3. The method according to claim 2, wherein said polymerase chain reaction mix is for real-time polymerase chain reaction.

4. The method according to claim 3, wherein said real-time polymerase chain reaction is a multiplex reaction.

5. The method according to any one of the preceding claims, wherein the presence of further bacteria is detected in the sample, said bacteria being selected from the group consisting of: *Streptococcus dysgalactiae, Streptococcus agalactiae, Streptococcus uberis, Escherichia coli, Enterococcus* spp., *Corynebacterium bovis, Arcanobacterium pyogenes, Klebsiella* spp., *Lactococcus lactis, Peptostreptococcus indolicats* and *Serratia* spp.

6. The method according to claim 1, wherein at least one of the primers specifically hybridizing with said nucleic acid sequence of clumping factor gene of *Staphylococcus aureus* are selected from the sequences consisting of: SEQ ID NO:1, SEQ ID NO:2 and SEQ ID NO:3.

7. The method according to claim 1, wherein at least one of the primers specifically hybridizing with said nucleic acid sequence of elongation factor Tu gene of *Staphylococcus* spp. are selected from the sequences consisting of: SEQ ID NO:4 and SEQ ID NO:5.

8. The method according to claim 2, wherein the probe specifically hybridizing with said nucleic acid sequence of clumping factor gene of *Staphylococcus aureus* has the sequence set forth in SEQ ID NO:6.

9. The method according to claim 2, wherein the probe specifically hybridizing with said nucleic acid sequence of elongation factor Tu gene of *Staphylococcus* spp. has the sequence set forth in SEQ ID NO:7.

10. The method according to claim 1, wherein the presence of β-lactamase production or a β-lactamase gene encoding resistance to β-lactam antibiotics is further detected in said sample.

11. Use of an oligonucleotide mix comprising SED ID NOS: 1-7 for the detection of the presence of *Staphylococcus aureus* and *Staphylococcus* spp. in a sample.

12. An oligonucleotide mix comprising SEQ ID NOS:1-7.

13. A kit for the detection of the presence of mastitis causing bacteria in a milk sample comprising a pair of primers specifically hybridizing with the nucleic acid sequence of clumping factor gene of *Staphylococcus aureus* and a pair of primers specifically hybridizing with the nucleic acid sequence of elongation factor Tu gene of *Staphylococcus* spp., wherein said primers specifically hybridizing with the nucleic acid sequence of clumping factor gene of *Staphylococcus aureus* amplify at least part of the 95bp long amplicon in said clumping factor gene corresponding to nucleotides 534-628 in SEQ ID NO:8; and said primers specifically hybridizing with the nucleic acid sequence of elongation factor Tu gene of *Staphylococcus* spp. amplify at least part of the 119bp long amplicon in said elongation factor Tu gene corresponding to nucleotides 603-721 in SEQ ID NO:9.

14. The kit according to claim 13 further comprising a probe specifically hybridizing with said nucleic acid sequence of clumping factor gene of *Staphylococcus aureus* and/or a probe specifically hybridizing with said nucleic acid sequence of elongation factor Tu gene of *Staphylococcus* spp.

15. The kit according to claim 14 for use in the real-time polymerase chain reaction.

16. The kit according to claim 13 or 14, wherein said kit comprises at least one of the primers and probes selected from the group consisting of SEQ ID NOS:1-7.

17. Use of a kit according to any one of claims 13-16 for the detection of the presence of mastitis causing bacteria in a milk sample.

18. Method for determining the presence of *Staphylococcus aureus* and *Staphylococcus* spp. in a sample comprising the steps of:
a) isolating nucleic acid from said sample;
b) contacting the isolated nucleic acid obtained in step a) in a polymerase chain reaction mix with primers specifically hybridizing with the nucleic acid sequence of clumping factor gene of *Staphylococcus aureus* and amplifying at least part of the 95bp long amplicon in said clumping factor gene corresponding to nucleotides 534-628 in SEQ ID NO:8 and with primers specifically hybridizing with the nucleic acid sequence of elongation factor Tu gene of *Staphylococcus* spp. and amplifying at least part of the 119bp long amplicon in said elongation factor Tu gene corresponding to nucleotides 603-721 in SEQ ID NO:9; and
c) performing a polymerase chain reaction with a reaction mix obtained from step b) so that the sequences of said clumping factor gene and elongation factor Tu gene are specifically amplified, if said sequences are present in the sample;
d) detecting the presence of the amplified nucleid acid sequences, wherein the presence of amplified nucleic acid sequence from both clumping factor gene and elongation factor Tu gene is indicative of the presence of *Staphylococcus aureus* in the sample, and wherein the presence of amplified nucleic acid sequence from only elongation factor Tu gene is indicative of the presence of *Staphylococcus* spp. in the sample.

## Patentansprüche

1. Verfahren zum Bestimmen des Vorhandenseins von Bakterien in einer Milchprobe, die Mastitis verursachen, das die Schritte umfasst:
a) das Isolieren von Nucleinsäuren aus einer Milchprobe oder einer von Milch abstammenden Probe;
b) das Inkontaktbringen der isolierten Nucleinsäuren, die in Schritt a) erhalten wurden, in einem Gemisch einer Polymerase-Kettenreaktion mit Primern, die spezifisch mit der Nucleinsäuresequenz des Clumping-Faktor-Gens von *Staphylococcus aureus* hybridisieren und das Amplifizieren von mindestens einem Teil des 95bp langen Amplikons in dem Clumping-Faktor-Gen, das den Nucleotiden 534 bis 628 von SEQ ID NO:8 entspricht und mit Primern, die spezifisch mit der Nucleinsäuresequenz des Elongationsfaktor-Tu-Gens von *Staphylococcus* spp. hybridisieren und das Amplifizieren von mindestens einem Teil des 119bp langen Amplikons in dem Elongationsfaktor-Tu-Gen, das den Nucleotiden 603 bis 721 von SEQ ID NO:9 entspricht; und
c) das Durchführen einer Polymerase-Kettenreaktion mit einem in Schritt b) erhaltenen Reaktionsgemisch, so dass die Sequenzen des Clumping-Faktor-Gens und des Elongationsfaktor-Tu-Gens spezifisch amplifiziert werden, falls die Sequenzen in der Probe vorhanden sind;
d) das Nachweisen des Vorhandenseins der amplifizierten Nucleinsäuresequenzen, wobei das Vorhandensein der amplifizierten Nucleinsäuresequenz von beiden, dem Clumping-Faktor-Gen und dem Elongationsfaktor-Tu-Gen, das Vorhandensein von *Staphylococcus aureus* in der Probe anzeigt, und wobei das Vorhandensein der amplifizierten Nucleinsäuresequenz nur des Elongationsfaktor-Tu-Gens das Vorhandensein von *Staphylococcus* spp. in der Probe anzeigt.

2. Verfahren nach Anspruch 1, wobei in Schritt b) die in Schritt a) erhaltene isolierte Nucleinsäure, zudem mit einer Sonde in Kontakt gebracht wird, die spezifisch mit der Nucleinsäuresequenz des Clumping-Faktor-Gens von *Staphylococcus aureus* hybridisiert und/oder einer Sonde, die spezifisch mit der Nucleinsäuresequenz des Elongationsfaktor-Tu-Gens von *Staphylococcus* spp. hybridisiert.

3. Verfahren nach Anspruch 2, wobei das Gemisch der Polymerase-Kettenreaktion für eine Echtzeit-Polymerase-Kettenreaktion (real-time PCR) ist.

4. Verfahren nach Anspruch 3, wobei die Echtzeit-Polymerase-Kettenreaktion eine Multiplex-Reaktion ist.

5. Verfahren nach einem der vorangegangenen Ansprüche, wobei das Vorhandensein von weiteren Bakterien in der Probe nachgewiesen wird, wobei die Bakterien ausgewählt sind aus der Gruppe bestehend aus: *Staphylococcus dysgalactiae, Streptococcus agalactiae, Streptococcus uberis, Escherichia coli, Enterococcus* spp., *Corynebacterium bovis, Arcanobacterium pyogenes, Klebsiella* spp., *Lactococcus lactis, Peptostreptococcus indolicus* und *Serratia* spp.

6. Verfahren nach Anspruch 1, wobei mindestens einer der Primer, der spezifisch mit der Nucleinsäuresequenz des Clumping-Faktor-Gens von *Staphylococcus aureus* hybridisiert, ausgewählt ist aus den Sequenzen bestehend aus: SEQ ID NO:1, SEQ ID NO:2 und SEQ ID NO:3.

7. Verfahren nach Anspruch 1, wobei mindestens einer der Primer, der spezifisch mit der Nucleinsäuresequenz des Elongationsfaktor-Tu-Gens von *Staphylococcus* spp. hybridisiert, ausgewählt ist aus den Sequenzen bestehend aus: SEQ ID NO:4 und SEQ ID NO:5.

8. Verfahren nach Anspruch 2, wobei die Sonde, die spezifisch mit der Nucleinsäuresequenz des Clumping-Faktor-Gens von *Staphylococcus aureus* hybridisiert, die in SEQ ID NO:6 dargelegte Sequenz hat.

9. Verfahren nach Anspruch 2, wobei die Sonde, die spezifisch mit der Nucleinsäuresequenz des Elongationsfaktor-Tu-Gens von *Staphylococcus* spp. hybridisiert, die in SEQ ID NO:7 dargelegte Sequenz hat.

10. Verfahren nach Anspruch 1, wobei das Vorhandensein der Herstellung von β-Lactamase oder eines β-Lactamase-Gens, das Resistenz gegen β-Lactam-Antibiotika codiert, zudem in der Probe nachgewiesen wird.

11. Verwendung eines Oligonucleotid-Gemischs, das SEQ ID NOs:1 bis 7 umfasst, zum Nachweis des Vorhandenseins von *Staphylococcus aureus* und *Staphylococcus* spp. in einer Probe.

12. Oligonucleotid-Gemisch, das SEQ ID NOs:1 bis 7 umfasst.

13. Kit zum Nachweis des Vorhandenseins von Bakterien in einer Milchprobe, die Mastitis verursachen, das ein Primer-Paar umfasst, das spezifisch mit der Nucleinsäuresequenz des Clumping-Faktor-Gens von *Staphylococcus aureus* hybridisiert und ein Primer-Paar, das spezifisch mit der Nucleinsäuresequenz des Elongationsfaktor-Tu-Gens von *Staphylococcus* spp. hybridisiert, wobei die Primer, die spezifisch mit der Nucleinsäuresequenz des Clumping-Faktor-Gens von *Staphylococcus aureus* hybridisieren, mindestens einen Teil des 95bp langen Amplikons in dem Clumping-Faktor-Gen amplifizieren, das den Nucleotiden 534 bis 628 von SEQ ID NO:8 entspricht, und die Primer, die spezifisch mit der Nucleinsäuresequenz des Elongationsfaktor-Tu-Gens von *Staphylococcus* spp. hybridisieren, mindestens einen Teil des 119bp langen Amplikons in dem Elongationsfaktor-Tu-Gen amplifizieren, das den Nucleotiden 603 bis 721 von SEQ ID NO:9 entspricht.

14. Kit nach Anspruch 13, das zudem eine Sonde umfasst, die spezifisch mit der Nucleinsäuresequenz des Clumping-Faktor-Gens von *Staphylococcus aureus* hybridisiert und/oder eine Sonde, die spezifisch mit der Nucleinsäuresequenz des Elongationsfaktor-Tu-Gens von *Staphylococcus* spp. hybridisiert.

15. Kit nach Anspruch 14 zur Verwendung in der Echtzeit-Polymerase-Kettenreaktion.

16. Kit nach Anspruch 13 oder 14, wobei das Kit mindestens einen der Primer und Sonden umfasst, die ausgewählt sind aus der Gruppe bestehend aus SEQ ID NOs:1 bis 7.

17. Verwendung eines Kits nach einem der Ansprüche 13 bis 16 zum Nachweis des Vorhandenseins von Bakterien in einer Milchprobe, die Mastitis verursachen.

18. Verfahren zum Bestimmen des Vorhandenseins von *Staphylococcus aureus* und *Staphylococcus* spp. in einer Probe, das die Schritte umfasst:
a) das Isolieren von Nucleinsäuren aus der Probe;
b) das Inkontaktbringen der isolierten Nucleinsäuren, die in Schritt a) erhalten wurden, in einem Gemisch einer Polymerase-Kettenreaktion mit Primern, die spezifisch mit der Nucleinsäuresequenz des Clumping-Faktor-Gens von *Staphylococcus aureus* hybridisieren und das Amplifizieren von mindestens einem Teil des 95bp langen Amplikons in dem Clumping-Faktor-Gen, das den Nucleotiden 534 bis 628 von SEQ ID NO:8 entspricht und mit Primern, die spezifisch mit der Nucleinsäuresequenz des Elongationsfaktor-Tu-Gens von *Staphylococcus* spp. hybridisieren und das Amplifizieren von mindestens einem Teil des 119bp langen Amplikons in dem Elongationsfaktor-Tu-Gen, das den Nucleotiden 603 bis 721 von SEQ ID NO:9 entspricht; und
c) das Durchführen einer Polymerase-Kettenreaktion mit einem in Schritt b) erhaltenen Reaktionsgemisch, so dass die Sequenzen des Clumping-Faktor-Gens und des Elongationsfaktor-Tu-Gens spezifisch amplifiziert werden, falls die Sequenzen in der Probe vorhanden sind;
d) das Nachweisen des Vorhandenseins der amplifizierten Nucleinsäuresequenzen, wobei das Vorhandensein der amplifizierten Nucleinsäuresequenz von beiden, dem Clumping-Faktor-Gen und dem Elongationsfaktor-Tu-Gen, das Vorhandensein von *Staphylococcus aureus* in der Probe anzeigt, und wobei das Vorhandensein der amplifizierten Nucleinsäuresequenz nur des Elongationsfaktor-Tu-Gens das Vorhandensein von *Staphylococcus* spp. in der Probe anzeigt.

## Revendications

1. Procédé de détermination de la présence de bactéries provoquant une mastite dans un échantillon de lait comprenant les étapes de :
a) isolation de l'acide nucléique à partir d'un échantillon de lait ou d'un échantillon dérivé du lait ;
b) mise en contact de l'acide nucléique isolé obtenu à l'étape a) dans un mélange de réaction en chaîne par polymérase avec des amorces s'hybridant de manière spécifique avec la séquence d'acides nucléiques du gène de facteur d'agglutination du staphylocoque doré et amplifiant au moins une partie de l'amplicon longue de 95bp dans ledit gène de facteur d'agglutination correspondant aux nucléotides 534 à 628 dans la séquence SEQ ID N° 8 et avec des amorces s'hybridant de manière spécifique avec la séquence d'acides nucléiques du gène de facteur d'élongation Tu du staphylocoque spp. et amplifiant au moins une partie de l'amplicon longue de 119 bp dans ledit gène de facteur d'élongation Tu correspondant aux nucléotides 603 à 721 dans la séquence SEQ ID N° 9 ; et
c) exécution d'une réaction en chaîne par polymérase avec un mélange de réaction obtenu à partir de l'étape b) de telle sorte que les séquences desdits gène de facteur d'agglutination et gène de facteur d'élongation Tu sont amplifiées de manière spécifique, si lesdites séquences sont présentes dans l'échantillon ;
d) détection de la présence des séquences d'acides nucléiques amplifiés, dans lesquelles la présence d'une séquence d'acides nucléiques amplifiés provenant à la fois du gène de facteur d'agglutination et du gène de facteur d'élongation Tu est représentative de la présence du staphylocoque doré dans l'échantillon, et dans lesquelles la présence de la séquence d'acides nucléiques amplifiés provenant uniquement du gène de facteur d'élongation Tu est représentative de la présence du staphylocoque spp. dans l'échantillon.

2. Procédé selon la revendication 1, dans lequel, dans l'étape b), ledit acide nucléique isolé obtenu dans l'étape a) est en outre mis en contact avec une sonde s'hybridant de manière spécifique avec ladite séquence d'acides nucléiques du gène de facteur d'agglutination du staphylocoque doré et/ou une sonde s'hybridant de manière spécifique avec ladite séquence d'acides nucléiques de gène de facteur d'élongation Tu du staphylocoque spp.

3. Procédé selon la revendication 2, dans lequel ledit mélange de réaction en chaîne par polymérase est destiné à une réaction en chaîne par polymérase en temps réel.

4. Procédé selon la revendication 3, dans lequel ladite réaction en chaîne par polymérase en temps réel est une réaction multiplex.

5. Procédé selon l'une quelconque des revendications précédentes, dans lequel la présence d'autres bactéries est détectée dans l'échantillon, lesdites bactéries étant sélectionnées à partir du groupe constitué par : *Streptococcus dysgalactiae, Streptococcus agalactiae, Streptococcus uberis, Escherichia coli, Enterococcus spp., Corynebacterium bovis, Arcanobacterium pyogenes, Klebsiella spp., Lactococcus lactis, Peptostreptococcus indolicus et Serratia spp..*

6. Procédé selon la revendication 1, dans lequel au moins l'une des amorces s'hybridant de manière spécifique avec ladite séquence d'acides nucléiques du gène de facteur d'agglutination du staphylocoque doré est sélectionnée à partir des séquences constituées par : la séquence SEQ ID N° 1, la séquence SEQ ID N° 2 et la séquence SEQ ID N° 3.

7. Procédé selon la revendication 1, dans lequel au moins l'une des amorces s'hybridant de manière spécifique avec ladite séquence d'acides nucléiques du gène de facteur d'élongation Tu du staphylocoque spp. est sélectionnée à partir des séquences constituées par : la séquence SEQ ID N° 4 et la séquence SEQ ID N° 5.

8. Procédé selon la revendication 2, dans lequel la sonde s'hybridant de manière spécifique avec ladite séquence d'acides nucléiques du gène de facteur d'agglutination du staphylocoque doré présente la séquence définie dans la séquence SEQ ID N° 6.

9. Procédé selon la revendication 2, dans lequel la sonde s'hybridant de manière spécifique avec ladite séquence d'acides nucléiques de gène de facteur d'élongation Tu du staphylocoque spp présente la séquence définie dans la séquence SEQ ID N° 7.

10. Procédé selon la revendication 1, dans lequel la présence d'une production de β-lactamase ou d'un gène de β-lactamase codant la résistance aux antibiotiques β-lactame est en outre détectée dans ledit échantillon.

11. Utilisation d'un mélange d'oligonucléotides comprenant les séquences SEQ ID N° 1 à 7 afin d'assurer la détection de la présence du staphylocoque doré et du staphylocoque spp. dans un échantillon.

12. Mélange d'oligonucléotides comprenant les séquences SEQ ID N° 1 à 7.

13. Kit de détection de la présence d'une bactérie provoquant la mastite dans un échantillon de lait comprenant une paire d'amorces s'hybridant de manière spécifique avec la séquence d'acides nucléiques du gène de facteur d'agglutination du staphylocoque doré et une paire d'amorces s'hybridant de manière spécifique avec la séquence d'acides nucléiques du gène de facteur d'élongation Tu du staphylocoque spp., dans lequel lesdites amorces s'hybridant de manière spécifique avec la séquence d'acides nucléiques du gène de facteur d'agglutination du staphylocoque doré amplifient au moins une partie de l'amplicon longue de 95 bp dans ledit gène de facteur d'agglutination correspondant aux nucléotides 534 à 628 dans la séquence SEQ ID N° 8 ; et lesdites amorces, s'hybridant de manière spécifique avec la séquence d'acides nucléiques du gène de facteur d'élongation Tu du staphylocoque spp., amplifient au moins une partie de l'amplicon longue de 119 bp dans ledit gène de facteur d'élongation Tu correspondant aux nucléotides 603 à 721 dans la séquence SEQ ID N° 9.

14. Kit selon la revendication 13, comprenant en outre une sonde s'hybridant de manière spécifique avec ladite séquence d'acides nucléiques du gène de facteur d'agglutination du staphylocoque doré et/ou une sonde s'hybridant de manière spécifique avec ladite séquence d'acides nucléiques du gène de facteur d'élongation Tu du staphylocoque spp.

15. Kit selon la revendication 14, destiné à une utilisation afin d'assurer une réaction en chaîne par polymérase en temps réel.

16. Kit selon la revendication 13 ou 14, dans lequel ledit kit comprend au moins l'une des amorces et sondes sélectionnées à partir du groupe constitué par les séquences SEQ ID N° 1 à 7.

17. Utilisation d'un kit selon l'une quelconque des revendications 13 à 16, afin d'assurer la détection de la présence de bactéries provoquant la mastite dans un échantillon de lait.

18. Procédé de détermination de la présence de staphylocoque doré et de staphylocoque spp. dans un échantillon comprenant les étapes de :
a) isolation de l'acide nucléique à partir dudit échantillon ;
b) mise en contact de l'acide nucléique isolé obtenu dans l'étape a) dans un mélange de réaction en chaîne par polymérase avec des amorces s'hybridant de manière spécifique avec la séquence d'acides nucléiques du gène de facteur d'agglutination du staphylocoque doré et amplifiant au moins une partie de l'amplicon longue de 95 bp dans ledit gène de facteur d'agglutination correspondant aux nucléotides 534 à 628 dans la séquence SEQ ID N° 8 et avec des amorces s'hybridant de manière spécifique avec la séquence d'acides nucléiques du gène de facteur d'élongation Tu du staphylocoque spp. et amplifiant au moins une partie de l'amplicon longue de 119 bp dans ledit gène de facteur d'élongation Tu correspondant aux nucléotides 603 à 721 dans la séquence SEQ ID N° 9 ; et
c) exécution d'une réaction en chaîne par polymérase avec un mélange de réaction obtenu à partir de l'étape b) de telle sorte que les séquences desdits gène de facteur d'agglutination et gène de facteur d'élongation Tu sont amplifiées de manière spécifique si lesdites séquences sont présentes dans l'échantillon ;
d) détection de la présence des séquences d'acides nucléiques amplifiées, dans lequel la présence de la séquence d'acides nucléiques amplifiés provenant à la fois du gène de facteur d'agglutination et du gène de facteur d'élongation Tu est représentative de la présence du staphylocoque doré dans l'échantillon, et dans lequel la présence de la séquence d'acides nucléiques amplifiés provenant du gène de facteur d'élongation Tu est représentative de la présence de staphylocoque spp. dans l'échantillon.
